# EUROPÄISCHE PATENTSCHRIFT

(11) **EP 1 202 675 B1**
(45) Veröffentlichungstag und Bekanntmachung des Hinweises auf die Patenterteilung: **06.04.2005**
(21) Anmeldenummer: 00906129.2
(22) Anmeldetag: 07.03.2000
(51) Int. Cl.: A61B 17/17, A61B 17/80

(54) **CHIRURGISCHER FÜHRUNGSKÖRPER**
SURGICAL GUIDE BODY
CORPS DE GUIDAGE CHIRURGICAL

(30) Priorität: 11.08.1999 DE 29913994 U
(43) Veröffentlichungstag der Anmeldung: 08.05.2002
(73) Patentinhaber: SYNTHES AG Chur, 7002 Chur (CH)
(72) Erfinder: FREI, Reto, CH-7270 Davos Platz (CH); HEHLI, Markus, CH-7276 Davos Frauenkirch (CH); DUDA, Georg, D-12209 Berlin (DE)
(74) Vertreter: Lusuardi, Werther Giovanni, Dr.
(86) Internationale Anmeldenummer: PCT/CH2000/000129
(87) Internationale Veröffentlichungsnummer: WO 2001/012081

(56) Entgegenhaltungen:
- EP-A- 0 558 789
- EP-A- 0 705 572
- EP-A- 0 723 764
- EP-A- 0 908 836
- WO-A-96/25892
- FR-A- 742 618
- FR-A- 2 254 298
- US-A- 3 741 205
- US-A- 5 476 467

## Beschreibung

Die Erfindung betrifft einen Führungskörper zur Aufnahme von in den Knochen zu verankernden, longitudinalen Fixationselementen gemäss dem Oberbegriff des Anspruchs 2 sowie einer Fixationsvorrichtung für die Knochenchirurgie mit einem Führungskörper gemäss dem Oberbegriff des Anspruchs 1.
Der Führungskörper kann als interner Fixator zur Osteosynthese wirken, z.B. am proximalen Humerus oder anderen gelenknahen Bereichen am Röhrenknochen.
Aus der US 5,476,467 BENOIST ist eine fächerartig gewellte Führungsschablone bekannt, um Kirschnerdrähte zu führen. Die Nachteile dieser Anordnung bestehen darin, dass die Fixationselemente (Kirschnerdrähte) lediglich parallel zueinander durch die Führungsschablone geführt werden können. Zudem besteht keine Möglichkeit durch Kirschnerdrähte nicht erreichbare Trümmer, bzw. Frakturteile mittels Fäden zu fixieren. Durch die gewellte Struktur der Schablone liegt diese nicht direkt auf dem Knochen auf, so dass die Länge der einzuführenden Drähte unnötig verlängert wird.
Aus der FR -A 2 254 298 CHATIN ist eine Knochenplatte bekannt, welche einen gebogenen Teil aufweist, in dessen Bohrungen frei rotierbare Zwischenstücke zur Aufnahme von Knochenschrauben angeordnet sind und welche einen geraden Stiel mit teilweise konischen Bohrungen aufweist. Eine windschiefe Anordnung der Bohrungsachsen ist jedoch nicht offenbart.

Hier will die Erfindung Abhilfe schaffen. Der Erfindung liegt das Problem zugrunde, einen Führungskörper für zur Aufnahme von in den Knochen zu verankernden longitudinalen Fixationselementen zu schaffen, welcher es erlaubt, Fixationselemente unter divergierenden Winkeln und sich gegenseitig kreuzend einzuführen. Der Führungskörper verhindert primär ein Wandern der intramedullär oder in der Spongiosa verlaufenden Drähte sowohl nach proximal als auch nach distal.

Die Erfindung löst die gestellte Aufgabe mit einem Führungskörper, welcher die Merkmale des Anspruchs 2 aufweist, sowie einer Fixationsvorrichtungen, welche die Merkmale des Anspruchs 1 aufweist.

Damit ist der Vorteil erzielbar, dass eine minimal invasive Operationstechnik angewendet werden kann, mit minimal zu inserierendem Implantatmaterial. Der erfindungsgemässe Führungskörper eignet sich insbesondere - wegen der Möglichkeit die Fixationselemente in drei Dimensionen anzuordnen - zur Osteosynthese am osteoporotischen oder durch Krankheit geschwächten Knochen. Die Stabilität der Osteosynthese wird somit primär durch die Bolzen/Drähte und ihre kreuzweise Positionierung im Knochen erbracht. Durch das direkte Anliegen des Führungskörpers am Knochen wird die freie Länge der einzuführenden Drähte auf ein Minimum reduziert. Dadurch wird eine frühe Belastung der Frakturstelle möglich und somit eine frühere Nutzung der betroffenen Gliedmassen und im Idealfall eine schnellere Heilung.

Eine bevorzugte Weiterbildung besteht darin, dass eine der Öffnungen des Führungskörpers ein Innengewinde aufweist, so dass auch Fixationselemente mit einen Aussengewinde einführbar sind.

Die Öffnungen des Führungskörpers weisen zweckmässigerweise einen Durchmesser im Bereich von 2 bis 6 mm auf.

Eine bevorzugte Weiterbildung besteht darin, dass der Führungskörper eine Anzahl zusätzlicher Löcher im Bereich des Randes des Führungskörpers aufweist, damit allfällige Knochenfrakturteile mittels Fäden an die Platte fixiert werden können. Als Randbereich wird eine Zone von nicht mehr als 10 mm Breite angesehen. Diese zusätzlichen Löcher sollten zweckmässigerweise keine scharfen Kanten aufweisen, damit sie die darin fixierten Fäden nicht verletzen können.
Die Anzahl solcher zusätzlicher Löcher, deren Durchmesser zwischen 1,5 und 2,5 mm liegen sollte, beträgt zweckmässigerweise 4 bis 6.

Bei einer weiteren bevorzugten Weiterbildung besteht der Führungskörper aus mehreren, übereinander geschichteter Gitter, vorzugsweise aus Metalldraht, welche durch einen Rahmen in ihrer relativen Lage festgehalten sind und deren übereinander liegenden Maschen die Öffnungen bilden. Durch die Gittermaschen lassen sich nun Kirschnerdrähte in frei wählbaren Winkeln und Positionen einbohren, wobei sie in ihrer relativen Lage durch die übereinander geschichteten Drahtgitter gehalten werden.

Vorzugsweise sollten die Kirschnerdrähte ein Gewinde aufweisen, damit eine Verrutschsicherung durch das Drahtgitter gegeben ist. Diese Weiterbildung hat den Vorteil, dass die Lage und Winkel der Kirschnerdrähte nicht vorgegeben ist und entsprechend der zu versorgenden Fraktur gewählt werden kann.

Vorzugsweise werden 2 bis 8 (typischerweise 4 bis 6) solcher Gitter direkt aufeinandergelegt, um eine minimale Bauhöhe des Implantats zu erreichen. Die Maschenweite der Gitter liegt vorzugsweise im Bereich von 1,5 bis 2,0 mm und sollte generell kleiner sein, als der Durchmesser der als Fixationselemente eingesetzten Kirschnerdrähte. Die einzelnen Gitter sollten so übereinander gelegt werden, dass nie zwei Gitter dieselbe Lage (Struktur) zueinander aufweisen. Der Verdrehwinkel zwischen den einzelnen Gitterlagen wird dementsprechend durch die Anzahl der eingesetzten Gitter bestimmt (z.B. 60° bei 6 Gittern). Die Drähte der einzelnen Gitter weisen vorzugsweise eine Dicke im Bereich von 0,2 bis 0,6 mm auf.

Die als Fixationsmittel eingesetzten Kirschnerdrähte sind vorzugsweise mit einem Aussengewinde versehen. Mindestens ein Teil der Öffnungen des Führungskörpers sollten ein Innengewinde aufweisen, welches mit dem Aussengewinde der Kirschnerdrähte korrespondiert. Das Fixationselement weist an seinem hinteren Ende vorzugsweise keinen Kopf auf und besitzt einen einheitlichen Durchmesser über die gesamte Länge gesehen. An seinem vorderen Ende, welches 10 bis 50 % der Gesamtlänge ausmachen kann, ist das Fixationselement vorzugsweise gewindelos ausgebildet. Das Fixationselement weist einen Durchmesser im Bereich von 2 bis 6 mm auf und die Maschenweite der Gitter sollte kleiner sein als der Durchmesser des Fixationselementes.

Eine bevorzugte Weiterbildung besteht darin, dass die Fixationsvorrichtung mit dem Führungskörper zusätzlich mindestens ein reib- oder formschlüssig in die Öffnungen des Führungskörpers einführbares, hohlzylindrisches Zwischenstück mit einer konzentrischen Bohrung aufweist. Das Fixationselement kann mittels Pressung, Klemmung oder Reibung im hohlzylindrischen Zwischenstück fixiert werden.

Die Erfindung und Weiterbildungen der Erfindung werden im folgenden anhand der teilweise schematischen Darstellungen mehrerer Ausführungsbeispiele noch näher erläutert.
Es zeigen:
Fig. 1 eine perspektivische Darstellung des Führungskörpers mit darin eingeführten Kirschnerdrähten;
Fig. 2 einen Querschnitt durch den Führungskörper gemäss Fig. 1 im Bereich eines darin eingeführten Kirschnerdrahtes;
Fig. 3 einen Querschnitt durch den Führungskörper im Bereich einer ihrer Öffnungen, einem in diese Öffnung einführbaren Zwischenstückes mit Gewinde und einem in das Zwischenstück einführbaren Fixationsmittel;
Fig. 4 einen Querschnitt durch den Führungskörper im Bereich einer ihrer Öffnungen, einem in diese Öffnung einführbaren Zwischenstückes ohne Gewinde und einem in das Zwischenstück einführbaren Fixationsmittel;
Fig. 5 eine perspektivische Darstellung eines Führungskörpers bestehend aus einem mehrlagigen Drahtgitter; und
Fig. 6 eine perspektivische Darstellung eines Führungskörpers bestehend aus einem mehrlagigen Drahtgitter und einem Schnabel zum Anbringen einer Befestigungsschraube.

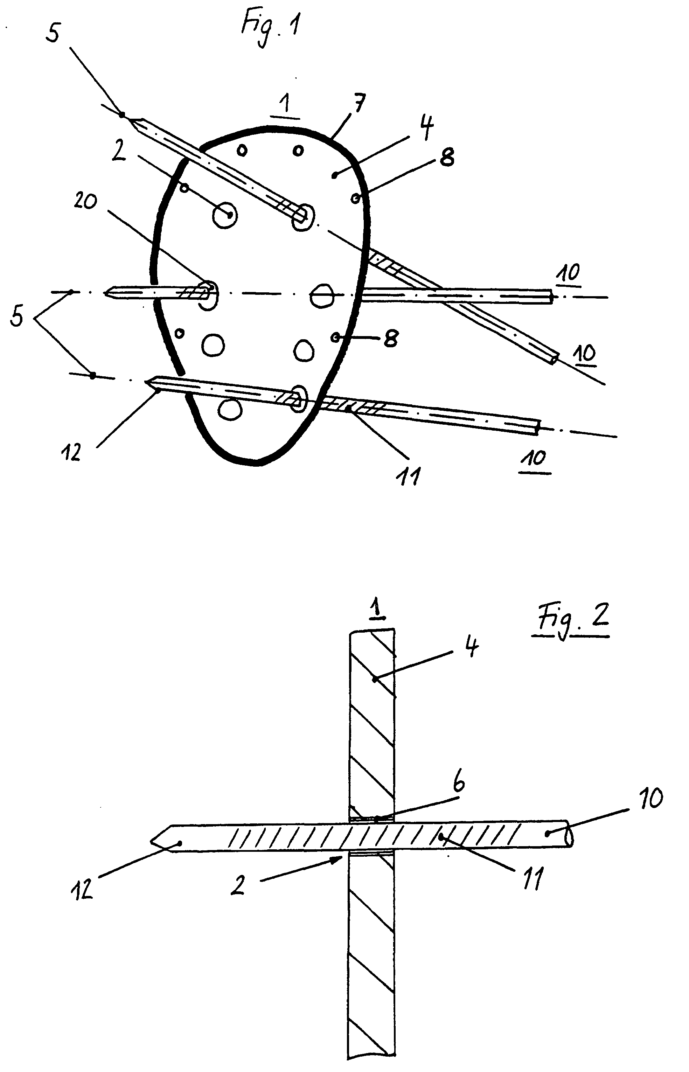

Der in Fig. 1 dargestellte Führungskörper 1 besteht aus einer ca. 2 bis 6 mm dicken, ebenen oder gewölbten Platte aus üblichen für die Implantation geeigneten metallischen Werkstoffen oder Kunststoffen (auch biodegradierbaren Kunststoffen) . Die Platte weist eine Vielzahl von Öffnungen 2 in Form von Bohrungen auf, welche einen Durchmesser von 2 bis 6 mm aufweisen und welche die Oberseite 3 mit der Unterseite 4 des Führungskörpers 1 verbinden. Der Mittelpunkt von mindestens drei dieser Öffnungen 2 liegen nicht auf einer Geraden. Durch die Öffnungen 2 lassen sich chirurgische Fixationselemente 10 wie Drähte, Nägel, Stifte oder Schrauben einführen, welche einen Durchmesser von 2 bis 6 mm aufweisen. In Fig. 1 sind dies Kirschnerdrähte mit einem Aussengewinde 11 und einem spitz ausgebildeten, vorderen Ende 12. Die zentralen Achsen 5 von mindestens zwei dieser Öffnungen 2 sind windschief zueinander angeordnet, damit die Fixationsmittel 10 dreidimensional angeordnet werden können. Die zentralen Achsen 5 der Öffnungen 2 schliessen vorzugsweise einen Winkel im Bereich von 50° bis 90° zur Ebene des plattenförmigen Führungskörpers 1 ein.
Am Rand 7 des Führungskörpers 1 sind zusätzlich sechs Löcher 8 für die weiter unten beschriebene Fadenfixation angebracht, welche einen Durchmesser von 2 mm aufweisen.
Die Unterseite 4 des Führungskörpers 1 ist vorzugsweise der zu applizierenden Knochenoberfläche angepasst, um eine möglichst gute und weitflächige Kontaktfläche zum Knochen zu erreichen.

Wie in Fig. 2 dargestellt, können die Öffnungen 2 in Form von Bohrungen mit einem Innengewinde 6 versehen sein, welches mit dem Aussengewinde 11 des Fixationsmittels 10 korrespondiert. Vorzugsweise weist das Fixationselement 10 an seinem hinteren Ende keinen Kopf auf, es können somit herkömmliche Kirschnerdrähte verwendet werden, so dass es einen einheitlichen Durchmesser über seine gesamte Länge aufweist. Im weiteren ist das Fixationselement 10 an seinem vorderen Ende 12, welches zehn bis fünfzig Prozent der Gesamtlänge des Fixationselementes 10 ausmacht, gewindelos ausgebildet. Das Gewinde ist lediglich zur Fixation des Fixationselementes 10 im plattenförmigen Führungskörper 1 notwendig, nicht jedoch für die Fixation im Knochen.

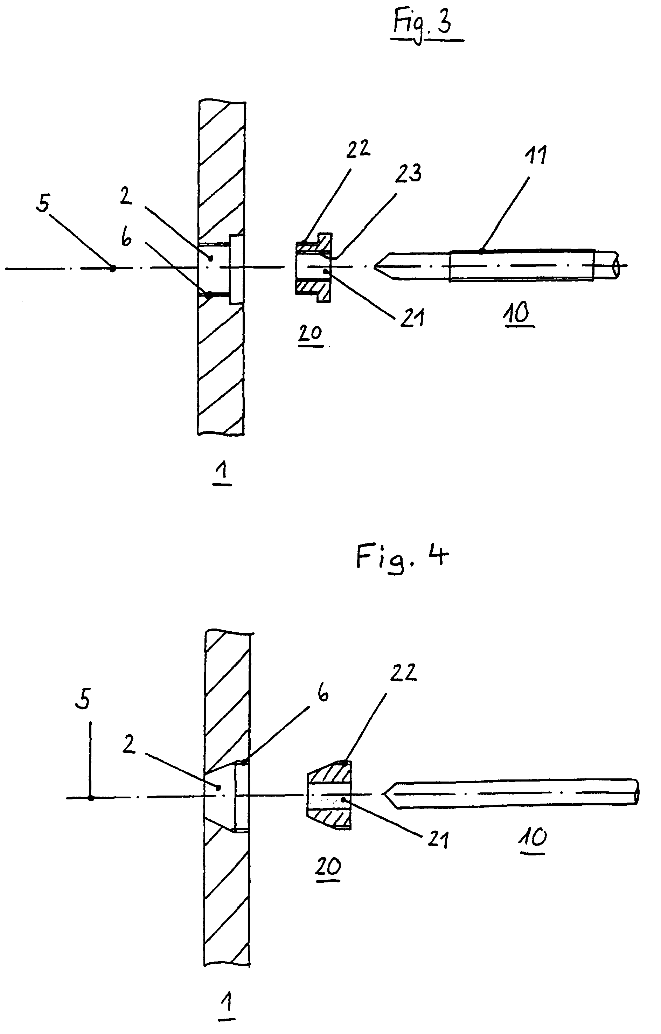

In Fig. 3 ist die bevorzugte Ausführung einer Fixationsvorrichtung mit einem Führungskörper 1 dargestellt, bei welcher ein reib- oder formschlüssig in die Öffnung 2 einführbares hohlzylindrisches (oder hohles konisches) Zwischenstück 20 mit einer konzentrischen Bohrung 21 vorgesehen ist. Das Zwischenstück 20 lässt sich bei entsprechender Passgenauigkeit einfach in die Öffnung 2 einführen, wo es durch die Reibungskraft gehalten wird. Es kann aber auch mit einem Aussengewinde 22 versehen werden, welches zum Innengewinde 6 der Öffnung 2 passt.
Das Fixationsmittel 10 kann dann statt direkt durch die Öffnung 2 durch die konzentrische Bohrung 21 des hohlzylindrischen Zwischenstückes 20 geführt werden. Im Inneren des Zwischenstückes 20 ist ein Innengewinde 23 vorgesehen, welches mit dem Aussengewinde 11 des Fixationsmittels 10 korrespondiert. Das Fixationsmittels 10 kann auch gewindelos ausgebildet sein und nur über eine radiale Klemmung durch das Zwischenstück 20 im Führungskörper 1 fixiert werden.

Bei einer weiteren in Fig. 4 dargestellten Ausführungsform ist das Zwischenstück 20 als konische Spann-/Klemmzange ausgebildet, welche reibschlüssig in die entsprechend konisch ausgebildete Öffnung 2 des Führungskörpers 1 einführbar ist, so dass keine Gewinde notwendig sind.

Der Führungskörper 1 lässt sich durch eine minimale Inzision in den Körper einführen, z.B. im Bereich des proximalen Humerus, an welchem es mit den Fixationsmitteln 10 befestigt werden kann. Die zusätzliche Verwendung von Knochenzement ist dabei nicht ausgeschlossen. Da die Platte über genügend Öffnungen 2 sowie über zusätzliche Löcher 8 im Bereich des Randes 7 des Führungskörpers 1 verfügt, die speziell zur Fixierung von Fäden ausgebildet sind, können diese dazu dienen Bänder an Knochenfragmenten des Humerus zu befestigen. Die dreidimensionale Anordnung der Fixationsmittel 10 verhindert deren Lockerung, so dass insgesamt eine stark verbesserte Stabilität der Fixation resultiert.

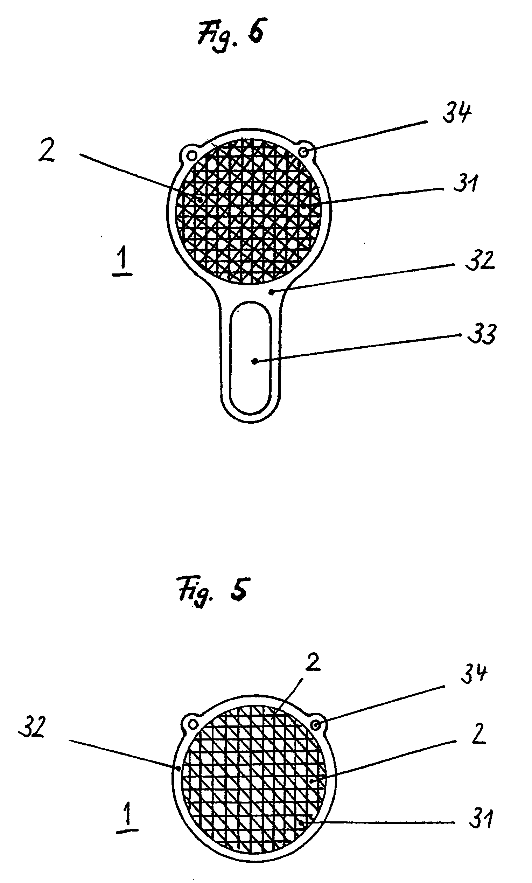

Bei einer weiteren in Fig. 5 dargestellten Ausführungsform des Führungskörpers 1 (hier in Form einer Führungsplatte) werden die Fixationselemente 10 durch ein vom Rahmen 32 gehaltenes, mehrlagiges Maschen-Gitter 31 in ihrer Lage und ihrem Winkel gehalten. Dabei werden die Fixationselemente 10 direkt durch das mehrlagige Gitter 31 hindurchgebohrt. Dies kann im gesamten Bereich des Durchmessers der einzelnen Öffnungen 2 geschehen. Die am Rahmen 32 angeordneten seitliche Löcher 34 dienen der möglichen Fixation des Führungskörpers 1 mittels Fäden.

Bei einer in Fig. 6 dargestellten Variante des Führungskörpers 1 gemäss Fig. 5 ist zusätzlich ein Langloch 33, welches auch ein normales, kreiszylindrisches Loch sein könnte, in Form eines Schnabels am Rahmen 32 angebracht. Es dient zur Aufnahme einer - zeichnerisch nicht dargestellten - Schraube, mit welcher der Führungskörper 1 am Knochen befestigt werden kann, bevor die Fixationselemente 10 durch den Führungskörper 1 in den Knochen eingebohrt werden.

Bei den Ausführungsformen gemäss den Fig. 5 und 6 weisen die zu verwendenden Fixationselemente 10 (typischerweise in Form von Kirschnerdrähten) ein Aussengewinde auf, um ein Verrutschen zu verhindern.

## Patentansprüche

1. Fixationsvorrichtung für die Knochenchirurgie mit einem Führungskörper (1) zur Aufnahme von in den Knochen zu verankernden longitudinalen Fixationselementen (10), wie Drähte, Nägel, Stifte oder Schrauben, wobei der Führungskörper (1) plattenförmig ausgebildet ist mit einer Oberseite (3), einer für den Knochenkontakt bestimmten Unterseite (4) sowie einer Anzahl die Oberseite (3) und Unterseite (4) des Führungskörpers (1) verbindender Öffnungen (2), durch welche chirurgische Fixationselemente (10) einführbar und im Knochen verankerbar sind, wobei die Fixationsvorrichtung mindestens ein reib- oder formschlüssig in die Öffnungen (2) des Führungskörpers (1) einführbares, zylindrisches oder konisches Zwischenstück (20) aufweist,
**dadurch gekennzeichnet, dass**
a) der plattenförmige Führungskörper (1) aus eine vollständig ebenen oder vollständig gewölbten Platte besteht;
b) die zentralen Achsen (5) von mindestens zwei der Öffnungen (2) windschief zueinander angeordnet sind;
c) das Zwischenstück (20) mit einer zur Zylinder- oder Konus-Achse des Zwischenstücks (20) konzentrischen Bohrung (21) ausgebildet ist; und
d) das Fixationselement (10) mittels Pressung, Klemmung oder Reibung im hohlzylindrischen Zwischenstück (20) fixierbar ist.

2. Führungskörper zur Aufnahme von in den Knochen zu verankernden longitudinalen Fixationselementen (10), wie Drähte, Nägel, Stifte oder Schrauben, wobei der Führungskörper (1) plattenförmig ausgebildet ist mit einer Oberseite (3), einer für den Knochenkontakt bestimmten Unterseite (4) sowie einer Anzahl die Oberseite (3) und Unterseite (4) des Führungskörpers (1) verbindender Öffnungen (2), durch welche chirurgische Fixationselemente (10) einführbar und im Knochen verankerbar sind, und wobei mindestens eine der Öffnungen (2) mindestens teilweise konisch ausgebildet ist,
**dadurch gekennzeichnet, dass**
a) der plattenförmige Führungskörper (1) aus einer vollständig ebenen oder vollständig gewölbten Platte besteht;
b) die zentralen Achsen (5) von mindestens zwei der Öffnungen (2) windschief zueinander angeordnet sind; und
c) das Fixationselement (10) mittels Pressung, Klemmung oder Reibung im hohlzylindrischen Zwischenstück (20) fixierbar ist.

3. Führungskörper (1) nach Anspruch 2, **dadurch gekennzeichnet, dass** er mit mindestens drei, die Oberseite (3) und Unterseite (4) des Führungskörpers verbindenden Öffnungen (2) versehen ist, deren Mittelpunkt nicht auf einer Geraden liegen.

4. Führungskörper (1) nach Anspruch 2 oder 3, **dadurch gekennzeichnet, dass** mindestens zwei der Öffnungen (2) zentrale Achsen (5) aufweisen, welche windschief zueinander verlaufen.

5. Führungskörper (1) nach einem der Ansprüche 2 bis 4, **dadurch gekennzeichnet, dass** mindestens eine der Öffnungen (2) ein Innengewinde (6) aufweist.

6. Führungskörper (1) nach einem der Ansprüche 2 bis 5, **dadurch gekennzeichnet, dass** die Öffnungen (2) einen Durchmesser im Bereich von 2 bis 6 mm aufweisen.

7. Führungskörper (1) nach einem der Ansprüche 2 bis 6, **dadurch gekennzeichnet, dass** eine Anzahl zusätzlicher Löcher (8) im Bereich des Randes (7) des Führungskörpers (1) vorgesehen sind, welche vorzugsweise einen geringeren Durchmesser als die Öffnungen (2) aufweisen.

8. Führungskörper (1) nach Anspruch 7, **dadurch gekennzeichnet, dass** die Anzahl der zusätzlichen Löcher (8) zwischen vier und sechs liegt.

9. Führungskörper (1) nach Anspruch 7 oder 8, dadurch gekenn zeichnet, dass der Durchmesser der zusätzlichen Löcher (8) zwischen 1,5 bis 2,5 mm liegt.

10. Führungskörper (1) nach einem der Ansprüche 2 bis 9, **dadurch gekennzeichnet, dass** er aus mehreren, übereinander geschichteter Gitter (31) besteht, welche durch einen Rahmen (32) in ihrer relativen Lage festgehalten sind und deren übereinanderliegenden Maschen die Öffnungen (2) bilden.

11. Führungskörper (1) nach Anspruch 10, **dadurch gekennzeichnet, dass** die Anzahl n der übereinander geschichteten Gitter (31) im Bereich von 2 < n < 8, vorzugsweise 4 < n < 6 liegt.

12. Führungskörper (1) nach Anspruch 10 oder 11, **dadurch gekennzeichnet, dass** die Maschenweite der Gitter (31) im Bereich von 1,5 bis 2,0 mm liegt.

13. Führungskörper (1) nach einem der Ansprüche 10 bis 12, **dadurch gekennzeichnet, dass** die einzelnen Gitter (31) gegeneinander verdreht angeordnet sind.

14. Führungskörper (1) nach einem der Ansprüche 10 bis 13, **dadurch gekennzeichnet, dass** die Drähte der einzelnen Gitter (31) eine Dicke im Bereich von 0,2 bis 0,6 mm aufweisen.

15. Fixationsvorrichtung für die Knochenchirurgie mit einem Führungskörper nach einem der Ansprüche 2 bis 14,
**dadurch gekennzeichnet, dass**
sie mindestens ein Fixationselement (10) umfasst.

16. Fixationsvorrichtung nach Anspruch 15, dadurch gekenn zeichnet, dass das Fixationselement (10) mit einem Aussengewinde (11) versehen ist.

17. Fixationsvorrichtung nach Anspruch 16, dadurch gekenn zeichnet, dass mindestens ein Teil der Öffnungen (2) ein Innengewinde (6) aufweisen, welches mit dem Aussengewinde (11) korrespondiert.

18. Fixationsvorrichtung nach einem der Ansprüche 15 bis 17, **dadurch gekennzeichnet, dass** das Fixationselement (10) an seinem hinteren Ende keinen Kopf aufweist.

19. Fixationsvorrichtung nach einem der Ansprüche 15 bis 18, **dadurch gekennzeichnet, dass** das Fixationselement (10) einen einheitlichen Durchmesser über die gesamte Länge gesehen aufweist.

20. Fixationsvorrichtung nach einem der Ansprüche 15 bis 19, **dadurch gekennzeichnet, dass** das Fixationselement (10) an seinem vorderen Ende (12) gewindelos ausgebildet ist.

21. Fixationsvorrichtung nach Anspruch 20, dadurch gekenn zeichnet, dass das vordere Ende (12) 10 bis 50 % der Gesamtlänge des Fixationselementes (10) ausmacht.

22. Fixationsvorrichtung nach einem der Ansprüche 15 bis 21, **dadurch gekennzeichnet, dass** das Fixationselement (10) einen Durchmesser im Bereich von 2 bis 6 mm aufweist.

23. Fixationsvorrichtung nach einem der Ansprüche 15 bis 22, **dadurch gekennzeichnet, dass** die Maschenweite der Gitter (31) kleiner ist als der Durchmesser des Fixationselementes (10).

24. Fixationsvorrichtung nach Anspruch 1 und einem der Ansprüche 15 bis 23.

25. Führungskörper nach einem der Ansprüche 2 bis 14, **dadurch gekennzeichnet, dass** er eine Dicke im Bereich von 2 - 6 mm aufweist.

## Claims

1. Fixation device for bone surgery including a guide body (1) intended to receive longitudinal fixation elements (10) such as wires, nails, pegs or screws to be anchored in the bone, whereby the guide body (1) is shaped in the form of a plate having a top surface (3), a bottom surface (4) intended for contact with the bone, and a number of openings (2) connecting the top surface (3) with the bottom surface (4) of the guide body (1), through which surgical fixation elements (10) are insertable and anchoreable in the bone, whereby the fixation device includes at least one cylindrical or conical connecting piece (20) which is insertable into the openings (2) of the guide body (1) in such a way as to be in frictional or positive engagement therewith,
**characterized in that**
a) the plate shaped guide body (1) consists of a entirely flat or entirely curved plate;
b) the central axes (5) of at least two openings (2) are in a skewed position relative to each other;
c) the connecting piece (20) is provided with a bore (21) being concentrically to the cylinder or cone axis of the connecting piece (20); and
d) the fixation element (10) may be kept in place within the hollow, cylindrical connecting piece (20) by means of press fit, force fit or friction.

2. A guide body (1) intended to receive longitudinal fixation elements (10) such as wires, nails, pegs or screws to be anchored within the bone, the guide body (1) being shaped in the form of a plate having a top surface (3), a bottom surface (4) intended for contact with the bone, and a number of openings (2) connecting the top surface (3) with the bottom surface (4) of the guide body (1), through which surgical fixation elements (10) are insertable and anchoreable in the bone, and whereby at least one of the openings (2) is configured at least partially conical,
**characterized in that**
a) the plate shaped guide body (1) consists of a entirely flat or entirely curved plate;
b) the central axes (5) at least two openings (2) are in a skewed position relative to each other; and
c) the fixation element (10) may be kept in place within the hollow, cylindrical connecting piece (20) by means of press fit, force fit or friction.

3. A guide body (1) as claimed in claim 1 or 2, **characterised in that** it is provided with at least three openings (2) which connect the top surface (3) with the bottom surface (4) of the guide body and the centres of which are not situated on a straight line.

4. A guide body (1) as claimed in any of the claims 1 to 3, **characterised in that** at least two of the openings (2) have central axes (5) which are skewed in relation to each other.

5. A guide body (1) as claimed in any of the claims 1 to 4, **characterised in that** at least one of the openings (2) is provided with an internal screw thread (6).

6. A guide body (1) as claimed in any of the claims 1 to 5, **characterised in that** the openings (2) have a diameter ranging between 2 and 6 mm.

7. A guide body (1) as claimed in any of the claims 1 to 6; **characterised in that** in the area of the edge portion (7) of the guide body (1) a number of additional holes (8) is included which preferably have a diameter smaller than that of the openings (2).

8. A guide body (1) as claimed in claim 7, **characterised in that** the number of additional holes (8) is between four and six.

9. A guide body (1) as claimed in claim 7 or 8, **characterised in that** the diameter of the additional holes (8) is between 1.5 and 2.5 mm.

10. A guide body (1) as claimed in any of the claims 1 to 9, **characterised in that** it consists of a plurality of grids (31) stacked on one another which are maintained in their relative positions by means of a frame (32) and the superimposed meshes of which form the openings (2).

11. A guide body (1) as claimed in claim 10, **characterised in that** the number n of the superimposed grids (31) is within a range of 2 < n < 8, preferably 4 < n < 6.

12. A guide body (1) as claimed in claim 10 or 11, **characterised in that** the mesh size of the grids (31) ranges between 1.5 and 2.0 mm.

13. A guide body (1) as claimed in any of the claims 10 to 12, **characterised in that** the individual grids (31) are in a twisted arrangement relative to one another.

14. A guide body (1) as claimed in any of the claims 10 to 13, **characterised in that** the wires forming the individual grids (31) have a thickness of between 0.2 and 0.6 mm.

15. A fixation device for bone surgery including a guide body as claimed in any of the claims 2 to 14,
**characterised in that**
it comprises at least one fixation element (10).

16. A fixation device as claimed in claim 15, **characterised in that** the fixation element (10) is provided with an external screw thread (11).

17. A fixation device as claimed in claim 16, **characterised in that** at least part of the openings (2) are provided with an internal screw thread (6) which corresponds to said external screw thread (11).

18. A fixation device as claimed in any of the claims 15 to 17, **characterised in that** the fixation element (10) is provided with a headless rear end portion.

19. A fixation device as claimed in any of the claims 15 to 18, **characterised in that** the fixation element (10) has a uniform diameter over its entire length.

20. A fixation device as claimed in any of the claims 15 to 19, **characterised in that** on its front end portion (12) the fixation element (10) is provided with a non-threaded portion.

21. A fixation device as claimed in claim 20, **characterised in that** said front end portion (12) corresponds to between ten and fifty percent of the total length of the fixation element (10).

22. A fixation device as claimed in any of the claims 15 to 21, **characterised in that** the fixation element (10) has a diameter ranging between 2 and 6 mm.

23. A fixation device as claimed in any of the claims 15 to 22, **characterised in that** the mesh size of the grids (31) is smaller than the diameter of the fixation element (10).

24. A fixation device as claimed in claim 1 and in any of the claims 15 to 23.

25. A guide body (1) as claimed in any of the claims 2 to 14, **characterised in that** it has a thickness in the range between 2 and 6 mm.

## Revendications

1. Dispositif de fixation utilisé en chirurgie osseuse avec un corps de guidage (1) permettant de recevoir des éléments de fixation longitudinaux (10) devant être ancrés dans l'os, tels que des fils métalliques, des clous, des broches ou des vis, le corps de guidage (1) étant réalisé en forme de plaque avec une face supérieure (3), une face inférieure (4) destinée à être en contact avec l'os ainsi qu'un nombre donné d'ouvertures (2) reliant la face supérieure (3) à la face inférieure (4) du corps de guidage (1) et à travers lesquelles des éléments de fixation chirurgicaux (10) peuvent être insérés, puis ancrés dans l'os, le dispositif de fixation présentant au moins une pièce intermédiaire (20) cylindrique ou conique pouvant être insérée par frottement ou par conjugaison de forme dans les ouvertures (2) du corps de guidage (1),
**caractérisé en ce que**
a) le corps de guidage (1) en forme de plaque se compose d'une plaque entièrement plane ou entièrement bombée;
b) les axes centraux (5) d'au moins deux des ouvertures (2) sont disposés de manière gauche l'un par rapport à l'autre;
c) la pièce intermédiaire (20) est formée avec un trou (21) concentrique à l'axe de la partie cylindrique ou conique de la pièce intermédiaire (20); et
d) l'élément de fixation (10) peut être fixé par pression, par serrage ou par frottement dans la pièce intermédiaire (20) en forme de cylindre creux.

2. Corps de guidage permettant de recevoir des éléments de fixation longitudinaux (10) destinés à être ancrés dans l'os, tels que des fils métalliques, des clous, des broches ou des vis, le corps de guidage (1) étant réalisé en forme de plaque avec une face supérieure (3), une face inférieure (4) destinée à être en contact avec l'os ainsi qu'un nombre donné d'ouvertures (2) reliant la face supérieure (3) et la face inférieure (4) du corps de guidage (1) et à travers lesquelles des éléments de fixation chirurgicaux (10) peuvent être insérés, puis ancrés dans l'os, et au moins une des ouvertures (2) étant réalisée au moins partiellement en forme de cône,
**caractérisé en ce que**
a) le corps de guidage (1) en forme de plaque se compose d'une plaque entièrement plane ou entièrement bombée;
b) les axes centraux (5) d'au moins deux des ouvertures (2) sont disposés de manière gauche l'un par rapport à l'autre;
c) l'élément de fixation (10) peut être fixé par pression, par serrage ou par frottement dans la pièce intermédiaire (20) en forme de cylindre creux.

3. Corps de guidage (1) selon la revendication 2, **caractérisé en ce qu'**il est pourvu d'au moins trois ouvertures (2) reliant la face supérieure (3) et la face inférieure (4) du corps de guidage et dont le centre ne se trouve pas sur une droite.

4. Corps de guidage (1) selon la revendication 2 ou 3, **caractérisé en ce qu'**au moins deux des ouvertures (2) présentent des axes centraux (5) s'étendant de manière gauche l'un par rapport l'autre.

5. Corps de guidage (1) selon l'une des revendications 2 à 4, **caractérisé en ce qu'**au moins une des ouvertures (2) présente un filet intérieur (6).

6. Corps de guidage (1) selon l'une des revendications 2 à 5, **caractérisé en ce que** les ouvertures (2) présentent un diamètre compris entre 2 et 6 mm.

7. Corps de guidage (1) selon l'une des revendications 2 à 6, **caractérisé en ce qu'**un nombre donné de trous supplémentaires (8) est prévu dans la zone du bord (7) du corps de guidage (1), lesquels présentent de préférence un diamètre inférieur à celui des ouvertures (2).

8. Corps de guidage (1) selon la revendication 7, **caractérisé en ce que** le nombre de trous supplémentaires (8) est compris entre quatre et six.

9. Corps de guidage (1) selon la revendication 7 ou 8, **caractérisé en ce que** le diamètre des trous supplémentaires (8) est compris entre 1,5 et 2,5 mm.

10. Corps de guidage (1) selon l'une des revendications 2 à 9, **caractérisé en ce qu'**il se compose de plusieurs grilles superposées (31), lesquelles sont maintenues dans leur position relative par un cadre (32) et dont les mailles superposées forment les ouvertures (2).

11. Corps de guidage (1) selon la revendication 10, **caractérisé en ce que** le nombre des grilles superposées (31) est compris entre 2 < n < 8, de préférence entre 4 < n < 6.

12. Corps de guidage (1) selon la revendication 10 ou 11, **caractérisé en ce que** la largeur des mailles des grilles (31) est comprise entre 1,5 et 2,0 mm.

13. Corps de guidage (1) selon l'une des revendications 10 à 12, **caractérisé en ce que** les différentes grilles (31) sont disposées les unes par rapport aux autres de manière décalée selon un angle donné.

14. Corps de guidage (1) selon l'une des revendications 10 à 13, **caractérisé en ce que** les fils métalliques constituant les différentes grilles (31) présentent une épaisseur comprise entre 0,2 et 0,6 mm.

15. Dispositif de fixation utilisé en chirurgie osseuse avec un corps de guidage selon l'une des revendications 2 à 14,
**caractérisé en ce qu'**
il comprend au moins un élément de fixation (10).

16. Dispositif de fixation selon la revendication 15, **caractérisé en ce que** l'élément de fixation (10) est pourvu d'un filet extérieur (11).

17. Dispositif de fixation selon la revendication 16, **caractérisé en ce qu'**au moins une partie des ouvertures (2) présente un filet intérieur (6) qui correspond au filet extérieur (11).

18. Dispositif de fixation selon l'une des revendications 15 à 17, **caractérisé en ce que** l'élément de fixation (10) ne présente pas de tête à son extrémité arrière.

19. Dispositif de fixation selon l'une des revendications 15 à 18, **caractérisé en ce que** l'élément de fixation (10) présente un diamètre uniforme sur l'ensemble de sa longueur.

20. Dispositif de fixation selon l'une des revendications 15 à 19, **caractérisé en ce que** l'élément de fixation (10) est dépourvu de filetage à son extrémité avant (12).

21. Dispositif de fixation selon la revendication 20, **caractérisé en ce que** l'extrémité avant (12) constitue entre 10 et 50% de la longueur totale de l'élément de fixation (10).

22. Dispositif de fixation selon l'une des revendications 15 à 21, **caractérisé en ce que** l'élément de fixation (10) présente un diamètre compris entre 2 et 6 mm.

23. Dispositif de fixation selon l'une des revendications 15 à 22, **caractérisé en ce que** la largeur des mailles des grilles (31) est inférieure au diamètre de l'élément de fixation (10).

24. Dispositif de fixation selon la revendication 1 et selon l'une des revendications 15 à 23.

25. Corps de guidage selon l'une des revendications 2 à 14, **caractérisé en ce qu'**il présente une épaisseur comprise entre 2 et 6 mm.
